**Europäisches Patentamt**

**European Patent Office**

**Office européen des brevets**

(19)

(11) Numéro de publication : **0 152 314 B2**

(12) # NOUVEAU FASCICULE DE BREVET EUROPEEN

(45) Date de publication du nouveau fascicule du brevet : **31.03.93 Bulletin 93/13**

(51) Int. Cl.⁵ : **B01J 23/80,** B01J 23/89, C01B 3/22

(21) Numéro de dépôt : **85400047.8**

(22) Date de dépôt : **11.01.85**

(54) **Procédé de fabrication de catalyseurs contenant du cuivre, du zinc et de l'aluminium, utilisables pour la production de méthanol à partir de gaz de synthése.**

(30) Priorité : **27.01.84 FR 8401256**

(43) Date de publication de la demande : **21.08.85 Bulletin 85/34**

(45) Mention de la délivrance du brevet : **12.08.87 Bulletin 87/33**

(45) Mention de la décision concernant l'opposition : **31.03.93 Bulletin 93/13**

(84) Etats contractants désignés : **BE DE GB IT NL SE**

(56) Documents cités :
    DE-A- 1 542 222
    DE-A- 2 056 612
    DE-A- 2 163 317
    DE-A- 2 950 251
    DE-B- 2 056 612
    FR-A- 2 113 467
    FR-A- 2 181 002
    FR-A- 2 441 420
    US-A- 3 388 972
    US-A- 3 923 694

(56) Documents cités :
    US-A- 3 988 263
    US-A- 4 111 847
    US-A- 4 279 781
    US-A- 4 283 581
    Ullmanns Enzyklopädie der technischen Chemie, Vol. 13 (1977) p. 562
    Gmelin, 1934 Al (B)31, p. 35

(73) Titulaire : **INSTITUT FRANCAIS DU PETROLE 4, Avenue de Bois Préau F-92502 Rueil-Malmaison (FR)**

(72) Inventeur : **Courty, Philippe 91, rue Condorcet F-78800 Houilles (FR)**
Inventeur : **Travers, Christine 15 ter, rue Beaumarchais F-92500 Rueil-Malmmaison (FR)**
Inventeur : **Durand, Daniel 18, rue Michelet F-92500 Rueil-Malmaison (FR)**
Inventeur : **Forestière, Alain Le Prévert Bt B Route de Lyon F-69390 Vernaison (FR)**
Inventeur : **Chaumette, Patrick 34, Côte de la Jonchère F-78380 Bougival (FR)**

EP 0 152 314 B2

Jouve, 18, rue Saint-Denis, 75001 PARIS

## Description

La présente invention concerne un procédé de préparation d'un catalyseur de composition essentiellement homogène, particulièrement actif, stable et sélectif, utilisable dans des procédés mettant en jeu une réaction équilibrée des oxydes de carbone (CO, $CO_2$) avec l'hydrogéne, en particulier la production de méthanol à partir de gaz de synthèse et les réactions de décomposition des alcools primaires et en particulier du méthanol ou de l'éthanol en un mélange contenant des oxydes de carbone et de l'hydrogéne.

Ces catalyseurs comprennent au moins trois métaux: cuivre, aluminium et zinc; ils peuvent en outre contenir éventuellement au moins un métal du groupe des terres rares, de numéros atomiques 57 à 71 inclus et/ou le zirconium; ils peuvent également contenir, en outre de 0,01 à 1% d'au moins un métal choisi dans le groupe formé par le palladium, le rhénium et le platine, et/ou de 0,5 à 5% d'argent.

Les catalyseurs à base d'oxydes de cuivre et de zinc sont connus depuis de nombreuses années; ils ont été décrits dès 1933 par DODGE (brevet US 1.908.696). Dans les brevets US 3.388.972, 3.546.140 et 3.790.505, la société américaine C.C.I. décrit l'utilisation de compositions ternaires Cu Zn Al pour la conversion à basse température du monoxyde de carbone (CO), et pour la synthése du méthanol. Dans le brevet français 2.113.467, la société SHELL préconise l'emploi de catalyseurs Cu Zn Dy pour la synthése du méthanol; Dy ou didyme représentant le mélange d'au moins deux oxydes des métaux des terres rares de numéros atomiques 57 à 71 inclus. Cette société montre que les meilleurs résultats correspondent à des catalyseurs contenant plusieurs oxydes des métaux des terres rares; l'emploi d'aluminium n'est pas suggéré.

Divers modes de préparation des catalyseurs Cu Zn Al sont décrits notamment dans le brevet US 3.923.694 (I.C.I.) et US 4.279.781 (United Catalysts).

L'ajout de métaux précieux, sous forme de chlorures, a été revendiquée entre autres, dans le brevet américain US 4.257.920 qui concerne des catalyseurs Cu Al Zn préparés par mélange de leurs oxydes et/ou carbonates, en présence d'un ciment alumineux, le catalyseur contenant en outre au moins un oxyde de métaux des terres rares, de numéros 57 à 71 inclus.

On peut remarquer que l'art antérieur ne suggère pas la mise en oeuvre de procédés dans lesquels on obtient un catalyseur essentiellement homogène, notamment pour la répartition des métaux Cu Zn Al. Par exemple, le brevet US 3.923.694 décrit une coprécipitation séquentielle où un précurseur de spinelle contenant de l'aluminium et du zinc est d'abord obtenu, puis où on coprécipite sur ce précurseur un composé Cu-Zn binaire. Dans de nombreux brevets, par exemple US 3.388.972, 3.546.140, FR 2.027.162, l'alumine composant essentiel du catalyseur, est incorporée à l'état d'oxyde ou, à l'état d'hydroxyde (US 4.279.781).

Dans le cas d'un catalyseur de synthèse de méthanol, il est nécessaire d'avoir dans le catalyseur activé et prêt à l'emploi, une concentration en métaux alcalins aussi faible que possible; les métaux alcalins proviennent en général de la coprécipitation; des teneurs inférieures à 0,1% et préférentiellement inférieures à 0,05% sont recommandées (US 3 923 694)

Or, si le procédé de précipitation à chaud et en présence de solutions diluées, décrit dans l'art antérieur et par exemple dans le brevet précité, conduit effectivement à des précurseurs hydratés, cristallisés au moins partiellement qui peuvent aisément être désalcalinisés par lavage, ces précurseurs, tout en contenant des phases divisées, ne sont pas homogènes, c'est-à-dire, ne présentent pas une composition identique en tout point à l'échelle 1-5 nanomètres. Le précurseur hydraté cristallisé au moins partiellement est un mélange d'au moins trois phases: une phase ternaire Cu Al Zn, avec $\dfrac{Cu + Zn}{Al} = 3$ (atomes, atome $^{-1}$), de type hydrotalcite; une phase binaire: la rosacite qui est un hydroxycarbonate mixte de cuivre et de zinc; un hydroxycarbonate de cuivre, la malachite, ainsi qu'éventuellement d'autres phases, comme par exemple le spinelle $Zn\,Al_2O_4$ décrit dans le brevet US 3.923.694. L'hétérogénéité de composition de ces catalyseurs a pour conséquence une activité, une sélectivité et une stabilité relativement faibles, même si, initialement ils contiennent un oxyde de cuivre bien dispersé, au moins en partie. On trouve dans diverses publications, par exemple dans F. TRIFIRO et al Preparation of Catalysts III p. 723-733 1983 Elsevier Science Publishers (Amsterdam), une description détaillée de la formation simultanée de ces phases.

Pour obtenir un précurseur hydraté homogène, il est nécessaire d'opérer la coprécipitation dans des conditions telles, que la vitesse de croissance des microcristaux soit beaucoup plus rapide que la vitesse de formation des germes microcristallins. On obtient alors une structure désordonnée, sans organisation cristalline particulière, mais d'une très grande homogénéité. Les conditions spécifiques à l'ensemble Cu Al Zn, plus éventuellement au moins un métal Ln du groupe des terres rares, de numéros 57 à 71 inclus et/ou du zirconium, et/ou de l'argent, et/ou du palladium sont explicitées ci-après.

Mais comme il a été dit plus haut, les catalyseurs de synthèse du méthanol, pour être performants, doivent aussi contenir une proportion de métaux alcalins aussi faible que possible.

Les catalyseurs préparés à partir de précurseurs hydratés amorphes, contiennent une proportion excessive de sodium, directement proportionnelle à la concentration initiale des solutions utilisées, correspondant à environ 0,1 à 0,2% en poids de sodium par rapport au poids de l'ensemble des métaux.

Or, il a été découvert que d'une façon surprenante, il était possible d'enlever les métaux alcalins sans modifier l'homogénéité du catalyseur par un protocole bien particulier, qui constitue l'un des objets de l'invention: le précipité humide est séché, activé thermiquement, puis broyé finement, mis en contact avec un excès de solution aqueuse acide (pH inférieur à 6,7), décanté, mis en suspension dans l'eau, séché par exemple par atomisation, puis si nécessaire mis en forme par tout procédé connu. On obtient ainsi un catalyseur particulièrement actif, sélectif et stable.

Les catalyseurs selon la présente invention contiennent avantageusement les métaux précités dans les proportions suivantes rapportées au poids total de métaux:
- cuivre: de 25 à 80% et préférentiellement de 45 à 70%
- zinc: de 10 à 50% et préférentiellement de 15 à 35%
- aluminium: de 4 à 25% et préférentiellement de 4 à 20%
- métaux des terres rares et/ou zirconium:
  jusqu'à 20% et préférentiellement de 2 à 18%

Ils peuvent éventuellement contenir, en outre, jusqu'à 1% en poids, par exemple et préférentiellement de 0,01 à 0,9% en poids d'au moins un métal choisi dans le groupe formé par Pd, Re, Pt, et/ou jusqu'à 5% en poids, par exemple et de préférence 0,5 à 5% d'argent métallique.

L'homogénéité de composition des catalyseurs à l'échelle du nanomètre peut être contrôlée par exemple par spectrométrie d'émission X dans un microscope électronique à balayage par transmission (MEBT) équipé d'un détecteur de rayons X du type silicium dopé couvrant la zone spatiale requise (par exemple de 1 à 20 keV pour les compositions revendiquées dans l'invention). Le mode opératoire est le suivant: un échantillon représentatif du catalyseur est broyé en une poudre fine (par exemple: granulométrie inférieure à 10 $\mu$ m) puis déposé sur une grille de microscopie électronique, le cas échéant après mise en suspension dans un solvant organique puis évaporation de ce dernier. Le matériau constituant la grille de microscopie électronique doit être choisi de manière à éviter d'éventuels problèmes d'interférences spectrales ou de signaux parasites (pour cette raison les grilles en cuivre ne sont pas utilisables).

Parmi les matériaux donnant des résultats satisfaisants, on peut citer: le nylon, le beryllium, le carbone.

Le microscope utilisé doit à la fois donner des images haute résolution (0,5 à 1 nm) dans le mode balayage (mode STEM dans la terminologie anglo-saxonne) et posséder une sensibilité élevée dans le mode microanalyse par émission X. Un appareil commercial tel que le MEBT Vacuum Generator HB 501 convient tout à fait (sensibilité limite meilleure que 1000 atomes d'un élément donné) pour déterminer l'échelle d'homogénéité du catalyseur.

Après sélection de la zone à analyser (typiquement 2 à 5 nm), on effectue simultanément plusieurs comptages, de durée 100-1000 s, conduisant à une statistique de comptage suffisamment précise (meilleure que 10%).

A partir des intensités mesurées sur les divers pics sélectionnés pour les divers éléments présents dans l'échantillon, on peut déterminer leurs concentrations relatives puis leurs rapports atomiques respectifs selon des techniques bien connues en émission X (voir par exemple REED S.J.B. Electron microprobe Analysis, Cambridge University Press 1975), pour chacune des particules constituant l'échantillon.

Les échantillons comparés doivent tous présenter la même épaisseur. Les valeurs moyennes des coefficients de correction sont les suivantes:

Coefficients de correction (ramenés à Cu-K$\alpha$ = 1).

| Mesure sur la raie | Elément | Coefficient |
|---|---|---|
| $K_\alpha$ | cuivre | 1 |
| $K_\alpha$ | aluminium | 4,86 |
| $K_\beta$ | zinc | 4,68 |

Ces coefficients ont été déterminés par le demandeur à partir d'oxydes mixtes calcinés à haute température Cu Al$_2$O$_4$, ZnAl$_2$O$_4$, Cu$_{1-x}$ Zn$_x$ Al$_2$O$_4$ (x = 0,25 - 0,50 - 0,75) constituant les échantillons de référence.

Le rapport atomique Zn/Cu sera calculé par exemple comme suit ($i_{K\beta}$Zn et $i_{K\alpha}$ Cu sont les intensités brutes moyennes sur plusieurs comptages).

Zn/Cu = 4,68 $i_{K\beta}$ Zn/$i_{K\alpha}$ Cu.

Les meilleurs résultats, en terme d'activité, de sélectivité et de stabilité sont obtenus avec des catalyseurs présentant une variation des rapports atomiques Cu/Zn et Cu/Al inférieure à 15% environ et préférentiellement inférieure à 10% environ par rapport à la valeur moyenne de ce rapport, à l'échelle de 50 Å (5 nanomètres).

Pour obtenir des catalyseurs homogènes, il est essentiel de préparer tout d'abord une solution (par définition, homogène) contenant le cuivre, le zinc et l'aluminium, ainsi qu'éventuellement au moins un élément des terres rares et/ou le zirconium et/ou éventuellement de l'argent et/ou du palladium, puis de transformer cette solution par une réaction de coprécipitation en une substance solide, appelée le précurseur du catalyseur et présentant toujours une très grande homogénéité de composition.

Les métaux Cu, Zn, Al ainsi qu'éventuellement les terres rares et/ou le zirconium et/ou l'argent et/ou le palladium, sont mis en oeuvre sous forme de composés solubles et de préférence solubles en milieu acide, bien que les complexes aminés (solubles en milieu ammoniacal) du cuivre, du zinc et du palladium puissent éventuellement être utilisés en addition au réactif alcalin de coprécipitation.

On utilisera par exemple les oxydes solubles (par exemple $Re_2O_7$), les hydroxydes, les carbonates, les hydroxycarbonates solubles en milieu acide (par exemple $Cu\,CO_3 \cdot Cu(OH)_2$, $ZnCO_3$, $Zn(OH_2)$, les nitrates, oxalates, tartrates, citrates, acétates, acétylacétonates ou encore des combinaisons anioniques telles que aluminate, ou perrhénate. Les nitrates sont les sels solubles qui sont le plus souvent mis en oeuvre.

Pour la préparation de ces masses catalytiques, il est essentiel d'utiliser des techniques de préparation permettant d'obtenir un produit de composition aussi homogène que possible, et évitant la ségrégation des différents éléments lors des différentes étapes unitaires de la préparation.

Un mode de préparation consiste à préparer, par au moins une réaction de coprécipitation (étape a), un précurseur hydraté, homogène, contenant les métaux Cu Zn Al et éventuellement au moins un métal des terres rares et/ou du zirconium et/ou de l'argent et/ou du palladium; la réaction de coprécipitation consiste à mettre en présence dans des conditions opératoires définies plus loin une solution de sels solubles des métaux Cu Zn Al, éventuellement terres rares et/ou du zirconium et/ou de l'argent et/ou du palladium avec une solution de carbonate et/ou d'hydrogénocarbonate et/ou d'hydroxyde de sodium et/ou de potassium de façon à obtenir un coprécipité qui, après lavage ultérieur (étape b), constitue le précurseur hydraté homogène contenant lesdits métaux au moins en partie sous forme d'hydroxycarbonates.

Toutes les techniques et appareillages qui ont été décrits dans l'art antérieur peuvent être utilisés ou appliqués à la réalisation de l'invention; on peut par exemple ajouter la solution de sels des métaux Cu Zn Al et autres dans la solution ou l'inverse. De préférence, on ajoutera les deux solutions, de façon simultanée, et en régulant leurs débits par le pH mesuré dans la zone réactionnelle, dans un réacteur comportant un système d'agitation efficace. D'une façon préférée, les deux solutions seront mises en contact dans la zone de turbulence maximum délimitée par le volume enveloppant l'appareillage d'agitation à l'intérieur du volume réactionnel.

On opère de préférence en continu, le temps de séjour moyen, exprimé en minutes et défini comme le rapport du débit volumique toral (litres/minute) des solutions injectées dans le réacteur, au volume dudit réacteur, exprimé en litres, peut par exemple varier de 0,1 à 30 minutes environ et de préférence de 5 à 12 minutes environ. Le volume utile du réacteur peut varier de quelques litres à environ 100 litres, et le produit de réaction est récupéré en continu, puis envoyé par exemple sur un filtre presse ou encore sur un filtre rotatif où il est lavé.

Un mode préféré de réalisation de l'invention consiste à faire réagir une solution de sels des métaux Cu Zn Al ainsi qu'éventuelléme nt au moins un sel soluble d'au moins un métal Ln où Ln désigne au moins un métal des terres rares, de numéros atomiques 57 à 71 inclus, ou le zirconium et/ou le palladium et/ou l'argent, portée à une température comprise entre 20 et 80°C environ et contenant 0,2 à 0,6 atome gramme environ de l'ensemble des métaux (Cu + Al + Zn + Ln + Pd + Ag) par litre avec une solution de carbonate et/ou d'hydrogénocarbonate et/ou d'hydroxyde de sodium et/ou de potassium à une température comprise entre 20 et 80°C environ et contenant 0,4 à 1,2 atomes gramme environ de cations alcalins par litre, la réaction de coprécipitation (étape a) étant menée entre 20 et 80°C environ, le pH mesuré dans le volume réactionnel étant régulé (ou fixé) à une valeur comprise entre 6,3 et 7,3 unités pH environ, et le temps de résidence du mélange (coprécipité + eaux mères) dans le volume réactionnel n'excèdent pas 12 minutes environ. Lorsque le cation est le sodium, cette solution peut contenir éventuellement, en outre, le rhénium sous forme d'anion perrhénate ($ReO_4^-$).

On obtient de la sorte un précurseur hydraté homogène, amorphe en diffraction X, donnant par diffraction X et enregistrement goniométrique un diagramme "plat". Ce produit est ensuite lavé (étape b) de façon à réduire sa teneur en métaux alcalins (exprimée en poids par rapport au poids total des métaux) à environ 0,1 - 0,3% en poids, et de préférence à environ 0,05 - 0,2% en poids.

L'étape c de préparation du catalyseur est constituée par un séchage et une activation thermique ou calcination telle que décrite ci-dessous.

Le séchage du coprécipité mixte homogénéisé peut être réalisé par tout procédé connu; on peut l'effectuer par exemple par atomisation (spray-drying). On obtient un produit homogène, en poudre calibrée contenant environ 60 à environ 80% poids d'oxydes potentiels. On peut également sécher le produit en étuve, par exemple entre environ 50 et environ 150°C, sous balayage d'air, de façon à ramener si nécessaire, la teneur en oxydes potentiels à environ 60 à 80% poids. Il est recommandé d'éviter la stagnation du précipité en présence de pressions partielles de vapeur d'eau proches de la pression de vapeur saturante à la température de séchage considérer, de tels traitements peuvent avoir pour conséquence une déshydratation partielle du précipité avec cristallisation d'oxyde cuivrique en gros cristallites.

L'activation thermique consiste à traiter le précipité séché, à une température d'environ 250 à environ 500°C et de préférence entre environ 300 et environ 380°C pendant une durée suffisante, par exemple pendant au moins 0,5 heure pour obtenir un catalyseur activé homogène ne renfermant pas plus de 12 % poids de matières volatiles (le taux de matières volatiles est mesuré par exemple par activation en présence d'air d'un poids donné de produit, placé dans une nacelle et calciné à 00-600°C pendant 4 heures).

L'activation thermique peut être menée par exemple en présence d'un gaz inerte contenant de 0 à 50% d'oxygène.

Les opérations de séchage et d'activation thermique peuvent enfin être combinées par utilisation de techniques de grillage-éclair ou de calcination par atomisation (spray-calcination), le produit étant alors pulvérisé dans un courant de gaz de combustion.

Le mélange d'oxydes activé thermiquement obtenu à l'étape (c) est alors éventuellement broyé de manière à obtenir un produit ayant une granulométrie inférieure à 0,5 mm environ et de préférence inférieure à 0,05 mm. Cette étape (d) de broyage n'est effectuée que si le mélange d'oxydes issu de l'étape (c) a une granulométrie supérieure à 0,5 mm.

Le produit broyé ou le mélange d'oxyde issu de l'étape (c) ayant une granulométrie inférieure à 0,5 mm environ et de préférence inférieure à 0,05 mm environ est alors désalcalinisé plus complètement (étape f) par lavage selon le mode opératoire suivant:

On forme une suspension aqueuse (étape e) contenant environ de 0,5 à environ 10 % en poids d'oxydes du mélange d'oxydes de granulométrie inférieure à 0,5 mm environ obtenu ci-dessus et de préférence contenant environ de 1,5 à environ 6 % en poids de ce mélange d'oxydes. La désalcalinisation est menée par brassage énergique de ladite suspension pendant un temps suffisant pour que le sodium ou le potassium se dissolvent dans la solution; la température de la solution est avantageusement comprise entre 15 et 45°C environ. La durée du lavage varie entre 0,2 et 1 heure environ. Le lavage peut aussi être mené en continu sur filtre à bande, ou encore par percolation; d'une façon préférée on utilisera pour le lavage une solution aqueuse diluée c'acide de préférence l'acide nitrique, présentant un pH inférieur à 6,7 environ et préférentiellement inférieur à 6 environ et avantageusement compris entre 2 et 5 environ. Au cours du lavage, le pH augmente et peut atteindre des valeurs supérieures à environ 7, ce qui traduit la désalcalinisation du solide.

Les opérations de broyage et de lavage acide de la suspension d'oxyde peuvent également être combinées en une seule opération unitaire, par exemple en effectuant un broyage humide en présence d'au moins une partie de la solution diluée d'acide.

La cinétique de désalcalinisation a été déterminée en suivant par dosages successifs la teneur en alcalin de la solution de lavage; une durée standard d'environ 30 minutes est en général suffisante pour amener la teneur en alcalin exprimée en poids d'alcalin par rapport au poids total des métaux au-dessous de 0,05 % et par exemple, d'une manière préférée, à environ 0,005 à 0,035 %.

Le catalyseur désalcalinisé, séparé des eaux de lavage par filtration (étape g) est alors séché (étape h), de préférence dans les conditions opératoires suivantes: la vitesse de séchage est telle que le temps de résidence du solide dans l'intervalle thermique 60-150°C ne soit pas supérieur à 10 secondes environ et, préférentiellement, secondes environ. Le temps de résidence est avantageusement compris entre 1 et 10 secondes et de préférence entre 1 et 5 secondes. Un mode opératoire préféré consiste à rajouter au précipité un minimum d'eau et à le sécher par atomisation (spray-drying); On obtient un produit homogène contenant de environ 60 à environ 80 % en poids d'oxydes et 40 à 20 % en poids d'eau. Tout autre mode opératoire permettant de satisfaire à cette exigence est éventuellement utilisable. Un appareil, par exemple de type rotatif, permettant de sécher en agitant est également utilisable. Par la vitesse de rotation, il y a formation d'une couche mince de produit qui est séché instantanément. Le temps de séjour dans l'appareil est de l'ordre de 10 secondes environ. Des temps de séjour supérieurs à 10 secondes entrainent une diminution de l'activité du catalyseur par recristallisation du cuivre.

Le catalyseur activé thermiquement (étape i) à une température comprise entre environ 250°C et environ 500°C peut ensuite être éventuellement mis en contact avec une solution aqueuse ou organique d'au moins un métal choisi dans le groupe formé par le palladium, le platine, le rhénium et/ou l'argent, de façon à disperser d'une manière essentiellement uniforme le dit métal, et à obtenir après reséchage et réactivation thermique comme ci-avant un catalyseur où le dit métal est bien dispersé (la dispersion peut par exemple se mesurer par chimisorption de gaz réactifs CO, $H_2$, sur le dit métal). A l'exception des halogénures et des sulfates, tout les sels solubles, par exemple nitrates, acétyl-acétonates, ainsi que les complexes par exemple nitrosamminés, amminés ou carbonylés sont utilisables.

La mise en forme du catalyseur est réalisée par tout procédé connu, par exemple par traitement du précipité humide (après dépôt des métaux additionnels), du précipité séché, du précipité activé thermiquement; les opérations de mise en forme par extrusion, dragéification, coagulation en gouttes sont utilisables; on peut éventuellement compacter le produit homogène activé thermiquement, le broyer par exemple en-dessous de 0,5 mm, le mélanger à raison de 0,5-5% de son poids avec au moins un composé adjuvant de pastillage choisi dans le groupe formé par le graphite, l'acide stéarique, les stéarates, et éventuellement un adjuvant de porosité choisi parmi la cellulose et les poudres d'origine végétale en contenant, les carbonates d'ammonium, les fibres textiles combustibles et le naphtalène, puis enfin, le pastiller en cylindres pleins de diamètres 2-6 mm ou en cylindres toriques de diamètres extérieur 3-6 mm et intérieur 1 à 4 mm et de hauteur 2 à 6 mm.

Le catalyseur mis en forme par pastillage subira éventuellement une ultime activation thermique dans les conditions opératoires précitees.

Le catalyseur activé thermiquement et prêt à l'emploi est constitué par une association très homogène d'oxydes. Dans cette association très homogène d'oxydes, les métaux et notamment le cuivre, le zinc et l'aluminium sont répartis à l'échelle de 5 nm de manière trés homogène, et les variations relatives des rapports atomiques Cu/Al, Cu/Zn, sont inférieures à 15% environ et préférentiellement à moins de 10% environ. La surface spécifique des dits catalyseurs varie entre environ 50 et environ 150 $m^2g^{-1}$.

Les conditions de mise en oeuvre des dits catalyseurs pour la fabrication du méthanol sont habituellement les suivantes: le catalyseur, chargé au réacteur, est tout d'abord préréduit par un mélange de gaz inerte (azote par exemple) et d'au moins un composé réducteur choisi dans le groupe formé par l'hydrogène, le monoxyde de carbone, les alcools et les aldéhydes en $C_1$ et $C_2$, le rapport molaire composé réducteur/ composé réducteur + gaz inerte étant de 0,001: 1 à 1: 1.

La température de réduction varie généralement de 100 à 300°C environ mais de préférence entre 140 et 260°C environ, la pression totale est habituellement comprise entre 0,1 et 10 MPa environ et de préférence entre 0,1 et 6 MPa environ; la vitesse volumétrique horaire est habituellement comprise entre $10^2$ et $4.10^4$ heure $^{-1}$ environ et de préférence entre $5.10^2$ et $10^4$ heure $^{-1}$ environ. (Température et Pression Normales(TNP)).

La réduction est d'abord menée, par exemple entre environ 140 et environ 160°C, en présence du mélange réducteur précité et avec un rapport molaire gaz réducteur/gaz réducteur + gaz inerte compris entre 0,001 et 0,1 environ et préférentiellement entre 0,005 et 0,05, environ pendant un temps suffisant pour que les concentrations en gaz réducteur soient les mêmes à l'entrée et à la sortie du réacteur (ce qui prouve que la première étape de réduction est terminée), il peut être avantageux dans une deuxième étape d'augmenter la température ainsi qu'éventuellement la concentration en gaz réducteur, et de poursuivre la réduction dans des conditions thermiques plus sévères.

La température de réduction varie alors entre environ 160 et environ 240°C, le rapport molaire gaz réducteur/gaz réducteur + gaz inerte est alors compris entre 0,01 et 1 environ, et préférentiellement entre 0,05 et 1 environ, les pressions et vitesse volumétrique horaire restant à l'intérieur des fourchettes précitées.

La préréduction du catalyseur sera préférentiellement menée en phase liquide si, par la suite, la réaction de synthése du méthanol se déroule en phase liquide.

La réaction proprement dite de synthèse du méthanol est réalisée dans les conditions opératoires suivantes: la pression est habituellement comprise entre 2 et 15 MPa environ et de préférence entre 4 et 15 MPa environ, le rapport molaire $H_2/2 CO + 3 CO_2$ est avantageusement compris entre 0,4 et 10 environ, mais de préférence entre 0,5 et 4 environ, lorsque la réaction est menée en phase gazeuse, et compris de préférence entre 0,5 et 1,5 environ lorsque la réaction est menée en phase liquide. La température est comprise entre 200 et 300°C environ, mais de préférence entre 220 et 270°C environ.

La vitesse volumique horaire (exprimée en volume TPN de mélange gazeux par volume de catalyseur et par heure) est usuellement comprise entre 1500 et 60.000 $h^{-1}$ environ et de préférence entre 2000 et 20.000 $h^{-1}$ environ.

Le catalyseur peut être utilisé en poudre fine calibrée (10-700 μm environ) ou en particules de diamètre équivalent 2 à 10 mm environ, en présence d'une phase gazeuse, ou d'une phase liquide (dans les conditions opératoires) et d'une phase gazeuse. La phase liquide peut être constituée par un ou plusieurs hydrocarbures ayant au moins 5 et de préférence au moins 10 atomes de carbone.

Dans ce mode de mise en oeuvre, il est préférable que les vitesses superficielles du gaz et du liquide, dans les conditions de température et de pression du procédé, soient d'au moins 1,5 cm/sec. environ et de préférence d'au moins 3 cm/sec. environ. Par vitesse superficielle on entend le rapport du débit volumique à la section du réacteur considéré vide de catalyseur.

Les conditions de mise en oeuvre des dits catalyseurs pour la décomposition des alcools primaires de $C_1$

6

à $C_5$ et en particulier du méthanol ou de l'éthanol sont habituellement les suivantes: le catalyseur, chargé au réacteur, est tout d'abord préréduit par un mélange de gaz inerte (azote par exemple) et d'au moins un composé réducteur choisi dans le groupe formé par l'hydrogène, le monoxyde de carbone, les alcools et les aldéhydes en $C_1$ et $C_2$, le rapport molaire composé réducteur/composé réducteur + gaz inerte étant de 0,001 : 1 à 1 : 1.

La température de réduction varie généralement de 100 à 300°C environ mais de préférence entre 140 et 260°C environ, la pression totale est habituellement comprise entre 0,1 et 10 MPa environ et de préférence entre 0,1 et 6 MPa environ; la vitesse volumétrique horaire est habituellement comprise entre $10^2$ et $4.10^4$ heure$^{-1}$ environ et de préférence entre $5.10^2$ et $10^4$ heure$^{-1}$ (Température et Pression Normales (TPN)).

La réduction est d'abord menée, par exemple entre environ 140 et environ 160°C, en présence du mélange réducteur précité et avec un rapport molaire gaz réducteur/gaz réducteur + gaz inerte compris entre 0,001 et 0,1 environ et préférentiellement entre 0,005 et 0,05 environ, pendant un temps suffisant pour que les concentrations en gaz réducteur soient les mêmes à l'entrée et à la sortie du réacteur (ce qui prouve que la première étape de réduction est terminée), il peut être avantageux dans une deuxième étape d'augmenter la température ainsi qu'éventuellement la concentration en gaz réducteur, et de poursuivre la réduction dans des conditions thermiques plus sévères.

La température de réduction varie alors entre environ 160 et environ 240°C, le rapport molaire gaz réducteur/gaz réducteur + gaz inerte est alors compris entre 0,01 et 1, et préférentiellement entre 0,05 et 1, les pressions et vitesse volumétrique horaire restant à l'intérieur des fourchettes précitées.

La réaction proprement dite de décomposition est réalisée dans les conditions opératoires suivantes: la pression est habituellement comprise entre 1 et 6 MPa environ et de préférence entre 2 et 5 MPa environ. La température est comprise entre 200 et 320°C environ, mais de préférence entre 220 et 300°C environ.

La vitesse volumique horaire de la charge (exprimée en litre de charge par litre de catalyseur et par heure) est usuellement comprise entre 0,1 et 5 h$^{-1}$ environ et de préférence entre 0,5 et 3 h$^{-1}$ environ.

Les exemples qui font suite décrivent différents aspects de l'invention, sans en limiter la portée.

## Exemple 1: (catalyseur A)

On dissout 241,6 g de nitrate cuivrique trihydraté (1 at.g Cu), 150 g de nitrate d'aluminium nonahydraté (0.4 at. g Al), 89,2 g de nitrate de zinc hexahydraté (0,3 at. g Zn) dans 3 litres d'eau bipermutée afin d'obtenir une solution (solution I) contenant 0,57 at. g de métaux par litre.

On dissout séparément 243,3 g de carbonate disodique dans 4 litres d'eau. On obtient une solution II contenant 1,15 at. g de sodium par litre.

La réaction est menée dans un réacteur de 1100 ml opérant en continu. Les ceux solutions I et II sont injectées simultanément dans le réacteur contenant au préalable 1 l d'eau à une température de 60°C à 70°C. La température est maintenue entre 60°C et 70°C pendant toute la durée de la précipitation. Le temps de résidence est de l'ordre de 10 min.

Les débits sont régulés par le pH qui varie entre 6,8 et 7,2 pendant toute la durée de la réaction. Le produit de la réaction est récupéré en continu dans un autre réacteur, filtré et lavé par trois fois 12 litres d'eau bipermutée. Le produit obtenu contient alors 25% en poids d'oxydes potentiels par rapport à son poids total. Le précipité est ensuite séché en étuve ventilée, circuit ouvert à 40°C pendant 16 heures puis à 90°C pendant 3 heures. Le produit sec obtenu contient alors 80% en poids d'oxydes potentiels par rapport à son poids total, et présente un diagramme plat en diffraction X caractéristique d'un composé amorphe. L'étude microscopique révèle une bonne homogénéité du produit. Le produit est ensuite activé thermiquement pendant 3 heures à 350°C sous air. La teneur en matière volatile est alors de 10 % en poids. Le produit ainsi obtenu contenant 0,17 % en poids de sodium est ensuite broyé à une granulométrie d'environ 0,1 mm puis désalcalinisé comme suit: 120 g d'oxydes sont mis en suspension dans 2,5 litres d'eau bipermutée, acidifiée par $HNO_3$ jusqu'à pH 4,5 et agités énergiquement pendant une heure pour favoriser la dissolution du sodium. Le produit est alors séparé des eaux de lavage par filtration, remis en suspension dans environ 250 cm$^3$ d'eau et séché par atomisation à 120° C pendant 5 secondes. La teneur en sodium exprimée en % en poids de métal par rapport au poids total des métaux du catalyseur est alors de 0,020 %. Ce catalyseur séché est alors calciné 3 heures à 350°C puis pastillé en cylindres pleins de diamètre 4 mm, après ajout de 2 % poids de graphite. Avant d'être chargé dans l'unité, le catalyseur A est activé thermiquement une dernière fois 2 heures à 350°C, la teneur en matières volatiles est alors de 3 % en poids par rapport au poids du catalyseur.

La surface spécifique développée est de l'ordre de 70 m$^2$ x g$^{-1}$. L'étude microscopique révèle une bonne homogénéité du produit.

Les rapports atomiques Cu/Zn varient très peu et sont compris entre 3,05 et 3,6 dans tout le volume du solide. La variation maximum ou rapport Cu/Zn est de l'ordre de 10 % par rapport à sa valeur moyenne.

Les rapports atomiques Cu/Al varient entre 2,3 et 2,7 dans tout le volume du solide. La variation maximum

du rapport Cu/Al est de l'ordre de 9 % par rapport à sa valeur moyenne.

Exemple 2: (comparaison - catalyseur A 1)

La préparation d'un catalyseur A 1 est effectuée selon la méthode de l'exemple 1 à l'exception du séchage après désalcalinisation qui est un séchage en étuve ventilée, circuit ouvert, durant 16 heures à 40°C, puis 3 heures à 120°C. Une analyse par diffraction des rayons X du produit obtenu, montre une hétérogénéité importante liée à la cristallisation partielle de l'oxyde de cuivre pendant l'étape de séchage. La suite des étapes, calcination et pastillage est identique à celle de l'exemple 1.

Exemple 3: (comparaison - catalyseur A 2).

La préparation d'un catalyseur A 2 est effectuée selon la méthode de l'exemple 1 à l'exception de la désalcalinisation qui est effectuée en milieu basique: le précipité est remis en suspension dans 2 litres d'eau bipermutée contenant 2 g de bicarbonate d'ammonium. Le pH de la solution de lavage ainsi obtenue est de 8,5. Après séparation des eaux de lavage par filtration, le précipité est remis en suspension dans environ 250 cm$^3$ d'eau bipermutée puis séché instantanément par atomisation. Les traitements thermiques et l'étape de pastillage sont identiques à ceux décrits dans l'exemple 1.

Exemple 4: (comparaison - catalyseur A 3).

Le catalyseur A 3 est préparé suivant le mode opératoire décrit dans l'exemple 1, mais sans l'étape de désalcalinisation. Le catalyseur obtenu contient 0,224% en poids de sodium résiduel par rapport au poids total des métaux.

Exemple 5: (catalyseur B)

On dissout 241,6 g de nitrate cuivrique, trihydraté (1 at. g Cu) 112,5 g de nitrate d'aluminium nonahydraté (0,3 at. g Al), 64,95 g de nitrate de lanthane hexahydraté (0,15 at. g La), 133,8 g de nitrate de zinc hexahydraté (0,45 at. g Zn) dans 3,5 litres d'eau afin d'obtenir une solution (solution I) contenant 0,54 at. g de métaux par litre.

On dissout séparément 272 g de carbonate disodique dans 4,5 litres d'eau. On obtient une solution II contenant 1,14 at. g de sodium par litre. Les différentes étapes de la préparation sont les mêmes que celles décrites dans l'exemple 1. La teneur en sodium du catalyseur est de 0,032% en poids par rapport au poids total des métaux.

Exemple 6:

Le catalyseur B 1 est préparé suivant le mode opératoire décrit dans l'exemple 5 à l'exception du fait que le précipité est désalcalinisé par deux lavages acides successifs. La teneur en sodium est alors de 0,010% en poids de sodium par rapport au poids total des métaux.

Exemple 7: (catalyseur C).

On dissout 241,6 g de nitrate cuivrique trihydraté (1 at.g Cu), 150 g de nitrate d'aluminium nonahydraté (0,4 at. g Al), 22 g de nitrate de cérium hexahydraté (0,05 at. g Ce), 104 g de nitrate de zinc (0,35 at. g Zn) héxahydraté dans 3,5 litres d'eau bipermutée afin d'obtenir une solution I contenant 0,51 at. g de métaux par litre.

On dissout séparément 257,6 g de carbonate disodique dans 4,2 litres d'eau bipermutée. La solution II obtenue contient 1,16 at. g de sodium par litre. Les opérations de précipitation, lavage, désalcalinisation, activation thermique et pastillage sont identiques à celles décrites dans l'exemple 1. La teneur en sodium du catalyseur final est de 0,030 % en poids par rapport au poids total des métaux.

Exemple 8: (catalyseur D).

On dissout 241,6 g de nitrate cuivrique trihydraté (1 at.g Cu), 168,75 g de nitrate d'aluminium nonahydraté (0,45 at. g Al), 104 g de nitrate de zinc hexahydraté (0,35 at. g Zn), 1,61 g de nitrate de palladium (0,007 at. g de Pd) dans 3,5 litres d'eau bipermutée. La solution I ainsi obtenue contient 0,52 at.g de métaux par litre.

On dissout séparément 259 g de carbonate disodique dans 4,2 litres d'eau afin d'obtenir une solution II contenant 1,16 at. g de sodium par litre. Les étapes de préparation sont identiques à celles décrites dans l'exemple 1.

Le catalyseur fini contient 0,75% en poids de palladium et 0,02% en poids de sodium par rapport au poids total des métaux.

Exemple 9: (comparaison - catalyseur E 1)

On précipite la solution I contenant les sels des métaux Cu, Al, Zn dans les proportions de l'exemple précédent à raison de 0,51 at. g de métaux par litre, par une solution de carbonate disodique contenant 1,16 at. g de sodium par litre. Le produit obtenu après précipitation, est lavé, calciné, désalcalinisé jusqu'à une teneur en sodium de 0,020% en poids par rapport au poids total des métaux, séché par atomisation, suivant les méthodes décrites dans l'exemple 1.

La teneur en oxyde est alors de 72 %. Ce produit est alors calciné 3 heures à 350° C.

Le palladium est déposé par malaxage de 100 g du produit calciné avec 150 cm³ de solution aqueuse contenant 1,49 g de chlorure de palladium dihydraté. La teneur en Pd métal est de 0,75 % en poids par rapport au poids total des métaux. La pâte épaisse est ensuite remise en suspension dans le minimum d'eau, puis séchée par atomisation à 120°C pendant 5 secondes. La teneur en oxydes est alors de 70 %. Ce produit est alors calciné 2 heures à 400°C, puis mis en forme suivant la méthode décrite dans l'exemple 1.

Exemple 10: (catalyseur E)

Le catalyseur E diffère du catalyseur E 1 décrit dans l'exemple 9 en ce que le palladium est déposé à l'aide d'une solution d'acétyl-acétonate de palladium Pd(C$_5$H$_7$O$_2$)$_2$ 100 g de précipité calciné sont malaxés avec 150 cm³ d'alcool éthylique contenant 2,130 g d'acétyl-acétonate de palladium. La teneur en Pd métal est de 0,75 % en poids par rapport au poids total des métaux. Le séchage, la calcination et la mise en forme sont les mêmes que celles décrites dans l'exemple 9 pour le catalyseur E 1.

Exemple 11: (catalyseur F).

On dissout 193,28 g de nitrate de cuivre trihydraté (0,8 at.g Cu), 4,25 g de nitrate d'argent (0,025 at. g Ag), 131,25 g de nitrate d'aluminium nonahydraté (0,35 at. g Al), 148,68 g de nitrate de zinc dihydraté (0,5 at. g Zn) dans 3,5 litres d'eau pour obtenir une solution I contenant 0,48 at. g de métaux par litre.

On dissout séparément 239,7 g de carbonate disodique dans 4 litres d'eau bipermutée. La solution II obtenue contient 1,13 at. g de sodium par litre. Les opérations de précipitation, lavage, désalcalinisation, activation thermique et pastillage sont les mêmes que celles décrites dans l'exemple 1. Les teneurs en sodium résiduel du catalyseur et en argent sont respectivement de 0,0196% et 2,82% en poids de métal par rapport au poids total des métaux.

Exemple 12: (catalyseur G).

On précipite la solution I contenant les sels des métaux Cu, Al, Zn dans les proportions de l'exemple 8, à raison de 0,51 at. g de métaux par litre, par une solution de carbonate disodique, contenant du perrhenate de sodium (NaReO$_4$) obtenu par dissolution de 257,6 g de carbonate disodique et de 1,172 g de NaReO$_4$ dans 4,1 litres d'eau distillée. La solution II obtenue contient 1,18 at. g de sodium par litre. Le produit obtenu après précipitation est lavé, calciné, désalcalinisé, atomisé, activé et mis en forme suivant les méthodes décrites dans l'exemple 1. Les teneurs en sodium résiduel et en rhénium du catalyseur, sont respectivement de 0,034% et 0,749% en poids par rapport au poids total des métaux.

Exemple 13: (catalyseur H).

On dissout 241,6 g de nitrate de cuivre, trihydraté (1 at.g Cu), 112,5 g de nitrate d'aluminium nonahydraté (0,3 at. g Al), 43,3 g de nitrate de lanthane hexahydraté (0,1 at. g La), 133,81 g de nitrate de zinc hexahydraté (0,45 at. g Zn) et 2,30 g de nitrate de palladium (0,01 at. g Pd) dans 3,5 litres d'eau bipermutée. On obtient une solution I contenant 0,53 at. g de métaux par litre.

Séparément on dissout 266,2 g de carbonate disodique dans 4,2 litres d'eau distillée, on obtient une solution II contenant 1,19 at. g de sodium par litre. Les opérations de précipitation, lavage, désalcalinisation, activation thermique et pastillage sont les mêmes que celles décrites dans l'exemple 1. Les teneurs en sodium résiduel, en palladium et en lanthane sont respectivement de 0,0256, 0,92 et 11,97% en poids de métal par rapport au poids total des métaux.

Exemple 14: (catalyseur I).

On dissout 241,6 g de nitrate cuivrique trihydraté (1 at.g Cu), 75 g de nitrate d'aluminium nonahydraté (0,2 at. g Al), 40 g de nitrate de praséodyme tétrahydraté (0,1 at. g Pr) et 163,55 g de nitrate de zinc hexahydraté (0,55 at. g Zn) dans 4 litres d'eau bipermutée. La solution I obtenue contient 0,46 at. g de métaux par litre.

On dissout séparément 264,7 g de carbonate disodique dans 4,5 litres d'eau bipermutée afin d'obtenir une solution II contenant 1,11 at. g de sodium par litre.

Les différentes étapes de la préparation du catalyseur sont ensuite les mêmes que celles décrites dans l'exemple 1. La teneur en poids de sodium résiduel par rapport au poids total des métaux est de 0,032%.

Exemple 15: (catalyseur K - Comparaison)

La préparation d'un catalyseur K est effectuée selon la méthode de l'exemple 1 à l'exception du broyage fin du produit calciné. Le produit obtenu après calcination contenant 0,17 % en poids de sodium est broyé très grossièrement à une granulométrie d'environ 2 mm. Le produit broyé est ensuite désalcalinisé, filtré, séché puis calciné suivant le processus décrit dans l'exemple 1. La teneur en sodium du produit final est de 0,07 % en poids de métal par rapport au poids total des métaux du catalyseur.

Exemple 16: (catalyseur L - Comparaison)

La préparation du catalyseur L est effectuée selon la méthode de l'exemple 1 à l'exception du fait que le séchage après désalcalinisation est un séchage par atomisation pendant une durée de 60 secondes à 120°C du produit désalcalinisé. Le mode d'activation est identique à celui de l'exemple 1.

Exemple 17: (catalyseur M - Comparaison)

La préparation du catalyseur M est effectuée selon la méthode de l'exemple 1 à l'exception du fait que le temps de résidence du précipité dans les eaux méres à l'étape (a) est de 45 minutes environ. Le produit obtenu dans ces conditions est partiellement cristallisé, comme le montre l'enregistrement goniométrique du diagramme de diffraction X. Toutes les étapes ultérieures sont identiques à celles décrites dans l'exemple 1.

Tous les catalyseurs décrits dans les exemples 1 à 17 sont testés en synthèse du méthanol en phase gazeuse.

Les catalyseurs sont préalablement réduits in situ par un mélange d'hydrogène et d'azote contenant 6% d'hydrogène dans l'azote, par paliers de températures successifs entre 160°C et 240°C à la pression atmosphérique.

Les conditions de test sont les suivantes:

température comprise entre 220°C et 270°C

pression 60 bars

vitesse volumique horaire 10.000 h$^{-1}$

rapport $H_2/2\ CO + 3\ CO_2 = 1,2$

Les performances catalytiques de ces catalyseurs sont décrites dans le tableau I ci-après. L'activité est exprimée en terme de productivité massique en kilogramme (Kg) de produit par Kg de catalyseur et par heure.

La sélectivité en méthanol exprimée en atome % de carbone par rapport à l'ensemble des oxydes de carbone est dans tous les cas comprise entre 99 et 100%.

Le tableau I résume les principales caractéristiques des diverses étapes de la préparation des catalyseurs.

La composition des catalyseurs est donnée dans le tableau 2 et les proportions sont données en pourcentage en poids de métal par rapport au poids total des métaux, dans le catalyseur. Les performances sont données après 100 heures de fonctionnement et après 1000 heures de fonctionnement. Ln désigne un métal du groupe des terres rares et/ou le zirconium M désigne un métal choisi dans le groupe formé par l'argent, le palladium, le platine et le rhénium.

Exemple 18:

Un litre de catalyseur A, mis sous forme de pastilles de hauteur 2 mm et de 2,4 mm de diamètre est testé dans une réaction de synthèse du méthanol en phase liquide, dans un réacteur de 4 cm de diamètre et de 3 mètres de hauteur.

La réduction du catalyseur est réalisée en phase gazeuse à pression atmosphérique entre 190° C et 240° C par paliers successifs par un mélange d'hydrogène et d'azote à 1 % d'hydrogène dans l'azote.

Après réduction du catalyseur, on injecte simultanément le solvant (coupe paraffinique de $C_{12}$ à $C_{18}$) à raison de 270 litres par heure et le gaz de synthèse sous une pression de 75 bars. Gaz et liquide circulent de haut en bas. Les conditions opératoires sont les suivantes:

pression 75 bars

température 250° C

rapport $H_2/ 2 CO + 3 CO_2 = 1$

vitesse volumique horaire 15800 $h^{-1}$

Dans ces conditions la productivité est de 0,54 Kg de méthanol par Kg de catalyseur et par heure. Cette production reste stable pendant plus de 2.000 h d'opération.

Exemple 19:

Le catalyseur B, décrit dans l'exemple 5, a été testé également pour la décomposition du méthanol. Le catalyseur est préalablement réduit par un mélange d'hydrogène et d'azote contenant environ 5,5% d'hydrogène par paliers successifs de températures entre 140°C et 260°C sous pression atmosphérique. La réaction proprement dite s'effectue à 290°C sous une pression de 30 bars, avec une vitesse volumique horaire liquide de 3 $h^{-1}$. La conversion du méthanol en un mélange contenant CO, $CO_2$ et $H_2$ est de l'ordre de 95% au bout de 100 h et de 94,5% au bout de 1.000 h de marche.

EP 0 152 314 B2

## TABLEAU 1

| Exemple | Cata- lyseur | Etape a | | | | Etape b | Etape d | Etape f | | Etape h | |
|---|---|---|---|---|---|---|---|---|---|---|---|
| | | Temps de résidence en min | pH | Précipité obtenu | | teneur en sodium % poids | granulométrie en mm | pH | teneur Na % poids | T° C | Temps |
| | | | | homogénéité | diagramme | | | | | | |
| 1 | A | 10 | 6,8-7,2 | homogène | plat | 0,17 % | 0,1 mm | 4,5 | 0,020 % | 120° C | 5 s |
| 2 | A$_1$ | 11 | 6,8-7,2 | homogène | plat | 0,18 % | 0,1 mm | 4,2 | 0,019 % | 40° C puis 120° | 16 h 3 h |
| 3 | A$_2$ | 10 | 6,8-7,2 | homogène | plat | 0,17 % | 0,1 mm | 8,5 | 0,150 % | 120° C | 5 s |
| 4 | A$_3^c$ | 10 | 7,0-7,3 | homogène | plat | 0,224 % | 0,1 mm | non réalisé | 0,224 % | 120° C | 5 s |
| 5 | B | 11 | 6,5-7,0 | homogène | plat | 0,15 % | 0,2 mm | 4,5 | 0,032 % | 110° C | 8 s |
| 6 | B$_1$ | 11 | 6,5-7,0 | homogène | plat | 0,15 % | 0,2 mm | 4,5** | 0,010 % | 110° C | 8 s |
| 7 | C | 10 | 6,8-7,2 | homogène | plat | 0,17 % | 0,1 mm | 4,5 | 0,030 % | 120° C | 5 s |
| 8 | D | 12 | 6,4-7,0 | homogène | plat | 0,16 % | 0,2 mm | 4,5 | 0,020 % | 110° C | 8 s |
| 9 | E$_1$ | 15 | 6,6-7,1 | homogène | plat | 0,18 % | 0,1 mm | 4,5 | 0,020 % | 140° C | 5 s |
| 10 | E | 15 | 6,6-7,1 | homogène | plat | 0,18 % | 0,1 mm | 4,5 | 0,020 % | 140° C | 5 s |
| 11 | F | 20 | 6,5-7,0 | homogène | plat | 0,17 % | 0,1 mm | 4,5 | 0,0196 % | 120° C | 5 s |
| 12 | G | 10 | 6,8-7,2 | homogène | plat | 0,20 % | 0,1 mm | 4,5 | 0,034 % | 110° C | 8 s |
| 13 | H | 20 | 6,8-7,2 | homogène | plat | 0,17 % | 0,15 mm | 4,5 | 0,0256 % | 120° C | 5 s |
| 14 | I | 10 | 6,8-7,2 | homogène | plat | 0,20 % | 0,1 mm | 4,5 | 0,032 % | 120° C | 5 s |
| 15 | K | 12 | 6,8-7,2 | homogène | plat | 0,17 % | 2 mm | 4,5 | 0,07 % | 120° C | 5 s |
| 16 | L | 10 | 6,8-7,2 | homogène | plat | 0,18 % | 0,1 mm | 4,5 | 0,02 % | 120° C | 60 s |
| 17 | M | 45 | 6,8-7,2 | -- | présente* des raies | 0,18 % | 0,1 mm | 4,5 | 0,02 % | 120° C | 5 s |

* le diagramme traduit l'existence d'un produit partiellement cristallisé.

** lavé 2 fois à pH = 4,5

TABLEAU 2

| Exemple | Catal. | Formule | Na% | Cu% | Al% | Zn% | Ln% | HC | Performance à t=100 h | | | Performance à t=1000h | | |
|---|---|---|---|---|---|---|---|---|---|---|---|---|---|---|
| | | | | | | | | | T°C | Pbars | Produc. | T°C | Pbars | Produc. |
| 1 | A | $Cu_1Al_{0,4}Zn_{0,3}Na_{0,817x10^{-3}}O_{1,9}$ | 0,020 | 67,64 | 11,48 | 20,86 | - | - | 240 | 60 | 0,70 | 245 | 60 | 0,70 |
| 2 | $A_1$ | $Cu_1Al_{0,4}Zn_{0,3}Na_{0,776x10^{-3}}O_{1,9}$ | 0,0190 | 67,64 | 11,48 | 20,86 | - | - | 270 | 60 | 0,50 | 280 | 60 | 0,45 |
| 3 | $A_2$ | $Cu_1Al_{0,4}Zn_{0,3}Na_{0,61x10^{-2}}O_{1,9}$ | 0,150 | 67,53 | 11,46 | 20,84 | - | - | 270 | 60 | 0,40 | 275 | 60 | 0,35 |
| 4 | $A_3$ | $Cu_1Al_{0,4}Zn_{0,3}Na_{0,91x10^{-2}}O_{1,9}$ | 0,224 | 67,48 | 11,46 | 20,83 | - | - | 270 | 60 | 0,35 | 280 | 60 | 0,25 |
| 5 | B | $Cu_1Al_{0,3}La_{0,15}Zn_{0,45}Na_{0,17x10^{-2}}O_{2,275}$ | 0,032 | 52,12 | 6,64 | 24,13 | 17,08 | - | 230 | 60 | 0,68 | 235 | 60 | 0,68 |
| 6 | $B_1$ | $Cu_1Al_{0,3}La_{0,15}Zn_{0,45}Na_{0,53x10^{-3}}O_{2,275}$ | 0,010 | 52,12 | 6,64 | 24,13 | 17,08 | - | 220 | 60 | 0,70 | 220 | 60 | 0,70 |
| 7 | C | $Cu_1Al_{0,4}Ce_{0,05}Zn_{0,35}Na_{0,168x10^{-2}}O_{2,05}$ | 0,030 | 60,95 | 10,35 | 21,94 | 6,72 | - | 230 | 60 | 0,70 | 235 | 60 | 0,68 |
| 8 | D | $Cu_1Al_{0,45}Zn_{0,35}Pd_{0,007}Na_{0,863x10^{-3}}O_{2,035}$ | 0,020 | 63,97 | 12,22 | 23,03 | - | 0,75 | 220 | 60 | 0,72 | 225 | 60 | 0,70 |
| 9 | $E_1$ | $Cu_1Al_{0,45}Zn_{0,35}Pt_{0,007}Na_{0,863x10^{-3}}O_{2,035}$ | 0,020 | 63,97 | 12,22 | 23,03 | - | 0,75 | 260 | 60 | 0,40 | 270 | 60 | 0,35 |
| 10 | E | $Cu_1Al_{0,45}Zn_{0,35}Pd_{0,007}Na_{0,863x10^{-3}}O_{2,035}$ | 0,020 | 63,97 | 12,22 | 23,03 | - | 0,75 | 225 | 60 | 0,70 | 230 | 60 | 0,68 |
| 11 | F | $Cu_{0,8}Ag_{0,025}Al_{0,35}Zn_{0,5}Na_{0,815x10^{-3}}O_{1,84}$ | 0,0196 | 53,13 | 9,86 | 34,16 | - | 2,82 | 225 | 60 | 0,70 | 225 | 60 | 0,68 |
| 12 | G | $Cu_1Al_{0,45}Zn_{0,35}Re_{0,004}Na_{0,14x10^{-2}}O_{2,03}$ | 0,034 | 63,98 | 12,22 | 23,03 | - | 0,749 | 230 | 60 | 0,68 | 240 | 60 | 0,64 |
| 13 | H | $Cu_1Al_{0,3}La_{0,1}Zn_{0,45}Pd_{0,01}Na_{0,13x10^{-2}}O_{2,28}$ | 0,0256 | 54,76 | 6,97 | 25,35 | 11,97 | 0,92 | 225 | 60 | 0,74 | 230 | 60 | 0,72 |
| 14 | I | $Cu_1Al_{0,2}Pr_{0,1}Zn_{0,55}Na_{0,165x10^{-2}}O_{2,03}$ | 0,032 | 53,39 | 4,53 | 30,21 | 11,84 | - | 235 | 60 | 0,70 | 240 | 60 | 0,66 |
| 15 | K | $Cu_1Al_{0,4}Zn_{0,3}La_{0,287x10^{-2}}O_{1,9}$ | 0,070 | 67,59 | 11,48 | 20,86 | - | - | 260 | 60 | 0,4 | 265 | 60 | 0,35 |
| 16 | L | $Cu_1Al_{0,4}Zn_{0,3}Na_{0,617x10^{-3}}O_{1,9}$ | 0,028 | 67,64 | 11,48 | 20,86 | - | - | 270 | 60 | 0,66 | 270 | 60 | 0,66 |
| 17 | M | $Cu_1Al_{0,4}Zn_{0,3}Na_{0,817x10^{-3}}O_{1,9}$ | 0,020 | 67,64 | 11,48 | 20,86 | - | - | 245 | 60 | 0,70 | 245 | 60 | 0,50 |
| 18 | A | $Cu_1Al_{0,4}Zn_{0,3}Na_{0,817x10^{-3}}O_{1,9}$ | 0,020 | 67,64 | 11,48 | 20,86 | - | - | 250 | 75 | 0,54 | 275 | 75 | 0,50 |

## Revendications

1. Procédé de préparation d'un catalyseur de composition essentiellement homogène contenant au moins trois éléments essentiels: le cuivre, le zinc et l'aluminium, caractérisé en ce qu'il comprend les étapes suivantes:

a) mise en contact d'une solution d'au moins un composé de métal alcalin choisi dans le groupe formé par les hydrogéno-carbonates, les carbonates et les hydroxydes avec une solution essentiellement homogène, contenant sous forme de sels et/ou de complexes solubles au moins l'ensemble des métaux cuivre, zinc et aluminium, ladite mise en contact ayant lieu à un pH régulé compris entre 6,3 et 7,3 environ, de manière à former un précipité homogène amorphe présentant un diagramme plat en diffraction X d'au moins les métaux cuivre, zinc et aluminium, le temps de résidence dudit précipité dans les eaux mères étant de 0,1 à 30 minutes,

b) lavage à l'eau du précipité obtenu à l'étape (a), jusqu'à réduire sa teneur en métaux alcalins à une valeur comprise entre 0,1 et 0,3 % environ en poids par rapport aux métaux exprimés en poids,

c) séchage puis calcination du précipité lavé obtenu à l'étape (b), de manière à le convertir en un mélange d'oxydes,

13

d) lorsque le mélange d'oxydes obtenu à l'étape (c) a une granulométrie supérieure à 0,5 millimètre, broyage dudit mélange d'oxydes à une granulométrie inférieure à 0,5 millimètre environ,

e) mise en suspension aqueuse du mélange d'oxydes de granulométrie inférieure à 0,5 millimètre environ,

f) lavage de la suspension d'oxydes obtenue à l'étape (e), par solution aqueuse d'un acide, ladite solution ayant un pH inférieur à 6,7 environ, de manière à réduire sa teneur en métaux alcalins à une valeur inférieure à environ 0,05 % en poids par rapport à l'ensemble des métaux,

g) séparation du mélange d'oxydes désalcalinisé obtenu à l'étape (f) des eaux de lavages,

h) séchage par atomisation du mélange d'oxydes désalcalinisé obtenu à l'étape (g) à une température de 60 à 150° C environ avec un temps de résidence compris entre 1 et 10 secondes, de manière à obtenir un produit ayant une teneur en oxydes d'environ 60 % à environ 80 % en poids.

i) activation thermique du mélange d'oxydes séché, essentiellement homogène, obtenu à l'étape (h).

**2.** Procédé de préparation d'un catalyseur selon la revendication 1, caractérisé en ce qu'il comprend les étapes suivantes:

a) mise en contact d'une solution d'au moins un composé de métal alcalin choisi dans le groupe formé par les hydrogéno-carbonates, les carbonates et les hydroxydes contenant de environ 0,4 à environ 1,2 atome-gramme par litre de cations de métaux alcalins, avec une solution essentiellement homogène, contenant sous forme de sels et/ou de complexes solubles au moins l'ensemble des métaux cuivre, zinc et aluminium à une concentration globale de environ 0,2 à environ 0,6 atome-gramme par litre, ladite mise en contact ayant lieu à un pH compris entre 6,3 et 7,3 environ et à une température comprise entre 20 et 80°C environ, de manière à former un précipité homogéne amorphe présentant un diagramme plat en diffraction X d'au moins les métaux cuivre, zinc et aluminium, le temps de résidence dudit précipité dans les eaux méres étant de 0,1 à 30 minutes,

b) lavage du précipité obtenu jusqu'à réduire sa teneur en métaux alcalins à une valeur comprise entre environ 0,05 et environ 0,2 % en poids par rapport aux métaux exprimés en poids,

c) séchage, puis calcination du précipité obtenu à l'étape (b) de manière à le convertir en un mélange d'oxydes ladite calcination ayant lieu à une température comprise entre 250° et 500°C environ,

d) lorsque le mélange d'oxydes obtenu à l'étape (c) a une granulométrie supérieure à 0,5 millimètre environ, broyage dudit melange d'oxydes à une granulométrie inférieure à 0,5 millimètre environ,

e) formation d'une suspension aqueuse contenant environ de 0,5 à environ 10% en poids d'oxydes du mélange d'oxydes de granulométrie inférieur à 0,5 mm environ,

f) lavage de la suspension d'oxydes obtenue à l'étape (e) par une solution aqueuse d'un acide, ladite solution ayant un pH inférieur à 6 environ, à une température de 15 à 45°C environ, de manière à réduire la teneur en métaux alcalins jusqu'à une valeur inférieure à environ 0,05 % en poids par rapport aux métaux,

g) séparation du mélange d'oxydes désalcalinisé, obtenu à l'étape (f), des eaux de lavages,

h) séchage par atomisation du mélange d'oxydes désalcalinisé, obtenu à l'étape (g), à une température de 60 à 150° C environ avec un temps de résidence compris entre 1 et 10 secondes environ, de manière à obtenir un produit ayant une teneur en oxydes d'environ 60 % à environ 80 % en poids,

i) activation thermique du mélange d'oxydes sec, essentiellement homogène, obtenu à l'étape (h), à une température comprise entre 250 et 500° C environ.

**3.** Procédé de préparation d'un catalyseur selon la revendication 1 ou 2, caractérisé en ce que le temps de résidence du précipité dans les eaux mères à l'étape (a) est inférieur à douze minutes environ.

**4.** Procédé selon l'une quelconque des revendications 1 à 3, caractérisé en ce que le mélange d'oxydes mis en suspension au cours de l'étape (e) à une granulométrie inférieure à environ 0,05 mm et on forme une suspension d'oxydes contenant de 1,5 à 6 % environ en poids d'oxydes.

**5.** Procédé selon l'une quelconque des revendications 1 à 4, caractérisé en ce que, au cours de l'étape (f), le lavage de la suspension d'oxydes est effectué par une solution diluée d'acide nitrique ayant un pH compris entre 2 et 5 environ jusqu'à obtention d'une teneur en métaux alcalins comprise entre environ 0,005 et environ 0,035 % en poids par rapport aux métaux et, au cours de l'étape (h), le séchage est effectué par atomisation pendant une durée comprise entre 1 et 5 secondes environ.

**6.** Procédé selon l'une quelconque des revendications 1 à 5, caractérisé en ce que, un ou plusieurs métaux additionnels choisis dans le groupe formé par les terres rares, le zirconium, l'argent, le palladium et le rhénium sont introduits dans le catalyseur au cours de l'étape (a).

7. Procédé selon l'une quelconque des revendications 1 à 6, caractérisé en ce que un ou plusieurs métaux additionnels choisis dans le groupe formé par le palladium, le platine, le rhénium et l'argent sont introduits par imprégnation du mélange d'oxydes obtenu à l'étape (i) à l'aide de sels et/ou de complexes de ces métaux ne contenant pas de soufre, ni d'halogène dans leurs compositions, le catalyseur étant ensuite séché par atomisation à une température de 60 à 150°C environ, puis réactivé thermiquement à une température comprise entre 250 et 500°C environ.

8. Procédé selon l'une des revendications 1 à 7, caractérisé en ce que les quantités de sels ou de complexes des métaux employés pour la formation du catalyseur sont telles que les proportions en poids de chaque élément métallique rapportées au poids total des métaux présents dans le catalyseur sont de:
   Cuivre = 25-80 %
   Zinc = 10-50 %
   Aluminium = 4-25 %
   Terres rares et/ou zirconium = 0-20 %
   Palladium = 0- 1 %
   Platine = 0- 1 %
   Rhénium = 0- 1 %
   Argent = 0- 5 %
   Métaux alcalins = 0- 0,05 %

9. Catalyseur de composition essentiellement homogène contenant au moins trois éléments essentiels : le cuivre, le zinc et l'aluminium,
   obtenu selon l'une des revendications 1 à 8, l'homogénéité étant
   telle que les variations des rapports atomiques Cu/Al et Cu/Zn sont inférieures à 15 % environ par rapport à la valeur moyenne desdits rapports à l'échelle de 50 Angströms (5nm), la surface spécifique du catalyseur étant de 50 à 150 $m^2.g^{-1}$ environ.

10. Catalyseur selon la revendication 9, caractérisé en ce qu'il contient au moins un métal choisi dans le groupe formé par les terres rares, le zirconium, le palladium, le rhénium et l'argent, les proportions en poids de chaque métal présent rapportées au poids total des métaux présents étant de:
    Zirconium = 2 - 18 %
    Terres rares = 2 - 18 %
    Palladium = 0,01 - 0,9 %
    Rhénium = 0,01 - 0,9 %
    Argent = 0,5 - 5 %

11. Utilisation d'un catalyseur selon l'une des revendications 9 ou 10, dans des procédés mettant en jeu une réaction équilibrée, comprenant d'un côté des oxydes de carbone (CO, $CO_2$) et de l'hydrogène et de l'autre côté un alcool.

12. Utilisation suivant la revendication 11, caractérisée en ce que l'on forme du méthanol à partir du mélange d'oxydes de carbone (CO, $CO_2$) et d'hydrogène à une température de 200 à 400°C environ sous une pression de 2 à 15 MPa environ, le rapport molaire $H_2/2CO + 3CO_2$ étant compris entre 0,4 et 10 environ.

13. Utilisation suivant la revendication 11, caractérisée en ce que l'on décompose un alcool primaire en un mélange contenant des oxydes de carbone (CO, $CO_2$) et de l'hydrogène à une température de 200 à 320° C environ sous une pression de 1 à 6 MPa environ.

**Patentansprüche**

1. Verfahren zur Herstellung einer im wesentlichen homogenen mindestens drei wesentliche Elemente: Kupfer, Zink und Aluminium enthaltenden Katalysatorzusammensetzung, dadurch gekennzeichnet, daß es folgende Verfahrensstufen umfaßt:
   a) Kontaktieren einer Lösung aus mindestens einer Alkalimetallverbindung, ausgewählt aus der Gruppe, enthaltend die Hydrogencarbonate, die Carbonate und die Hydroxide, mit einer im wesentlichen homogenen Lösung, enthaltend in Form von Salzen und/oder löslichen Komplexen mindestens die Gesamtheit der Metalle Kupfer, Zink und Aluminium, wobei dieses Kontaktieren bei einem ph-Wert, der zwischen etwa 6,3 und 7,3 eingestellt wird, derart stattfindet, daß ein homogenes, amorphes Präpizitat, welches ein flaches Röntgenbeugungsdiagramm aus mindestens den Metallen Kupfer, Zink und Aluminium aufweist, gebildet wird und die Verweildauer des Präzipitats in den Mutterlaugen 0,1 bis 30 Minuten beträgt,

b) Waschen des aus Stufe (a) erhaltenen Präzipitats mit Wasser, bis sein Gehalt an Alkalimetallen auf einen Wert zwischen etwa 0,1 und 0,3 Gew.-%, bezogen auf das Metallgewicht, verringert ist.

c) Trocknung und anschließendes Kalzinieren des aus Stufe (b) erhaltenen gewaschenen Präzipitates, derart, daß es in eine Oxidmischung umgewandelt wird,

d) weist die Stufe (c) erhaltene Mischung aus Oxiden eine Korngröße oberhalb von 0,5 mm auf, erfolgt eine Zerkleinerung dieser Oxidmischung bis zu einer Korngröße von weniger als etwa 0,5 mm,

e) Erstellen einer wässrigen Suspension aus der Mischung von Oxiden mit einer Korngröße von weniger als etwa 0,5 mm,

f) Waschen der aus Stufe (e) erhaltenen Suspension von Oxiden mit einer wässrigen Lösung einer Säure, wobei diese Lösung einen pH-Wert von weniger als etwa 6,7 aufweist, so daß eine Verringerung ihres Gehalts an Alkalimetallen auf einen Wert unterhalb von etwa 0,05 Gew.-%, bezogen auf die Metalle insgesamt, erhalten wird,

g) Trennung der aus Stufe (f) erhaltenen dealkalisierten Mischung aus Oxiden vom Waschwasser,

h) Sprühtrocknung der aus Stufe (g) erhaltenen dealkalisierten Mischung aus Oxiden bei einer Temperatur von etwa 60 bis 150°C und einer Verweilzeit zwischen 1 bis 10 Sekunden, so daß ein Produkt mit einem Gehalt an Oxiden von etwa 60 bis 80 Gew.-% erhalten wird.

i) Thermische Aktivierung der aus Stufe (h) erhaltenen getrockneten, im wesentlichen homogenen Mischung aus Oxiden.

2. Verfahren zur Herstellung eines Katalysators nach Anspruch 1, dadurch gekennzeichnet, daß folgende Verfahrensstufen enthalten sind:

a) Inkontaktbringen einer Lösung aus mindestens einer Alkalimetallverbindung, ausgewählt aus der Gruppe, gebildet durch die Hydrogencarbonate, die Carbonate und die Hydroxide, enthaltend etwa 0,4 bis etwa 1,2 Grammatom pro Liter Alkalimetallkationen, mit einer im wesentlichen homogenen Mischung, die in Form von Salzen und/oder von löslichen Komplexen mindestens die Gesamtheit der Metalle Kupfer, Zink und Aluminium in einer Gesamtkonzentration von etwa 0,2 bis etwa 0,6 Grammatom pro Liter enthält, wobei dieses Inkontaktbringen bei einem pH-Wert zwischen etwa 6,3 und 7,3 und einer Temperatur im Bereich zwischen etwa 20° und 80°C derart stattfinden läßt, daß sich ein homogenes amorphes Präzipitat mit einem flachen Röntgenbeugungsdiagramm aus mindestens den Metallen Kupfer, Zink und Aluminium bildet, und die Verweilzeit dieses Präzipitats in den Mutterlaugen 0,1 bis 30 Minuten beträgt,

b) Waschen des erhaltenen Präzipitates bis sein Gehalt an Alkalimetallen auf einen Wert zwischen etwa 0,05 bis etwa 0,2 Gew.-%, bezogen auf das Metallgewicht, verringert ist,

c) Trocknung, dann Kalzinierung des aus Stufe (b) erhaltenen Präzipitates, so daß es in eine Mischung aus Oxiden umgewandelt wird, wobei die Kalzinierung bei einem Temperaturbereich zwischen etwa 250° und 500°C stattfindet,

d) weist die aus Stufe (c) erhaltene Mischung aus Oxiden eine Teilchengröße von mehr als etwa 0,5 mm auf, erfolgt eine Zerkleinerung dieser Mischung aus Oxiden bis zu einer Teilchengröße von weniger als etwa 0,5 mm,

e) Bildung einer wässrigen Suspension, die etwa 0,5 bis etwa 10 Gew.-% Oxide mit einer Teilchengröße von weniger als 0,5 mm aus einer Oxidmischung enthält,

f) Waschen der aus Stufe (e) erhaltenen Suspension aus Oxiden mit einer wässrigen Lösung einer Säure, wobei diese Lösung einen pH-Wert von weniger als 6 aufweist, bei einer Temperatur von etwa 15 bis 45°C, so daß der Gehalt an Alkalimetallen bis auf einen Wert von weniger als 0,05 Gew.-%, bezogen auf die Metalle, verringert wird,

g) Trennung der aus Stufe (f) erhaltenen dealkalisierten Mischung aus Oxiden vom Waschwasser,

h) Sprühtrocknung der aus Stufe (g) erhaltenen dealkalisierten Mischung aus Oxiden bei einer Temperatur von etwa 60 bis 150°C und einer Verweildauer zwischen etwa 1 und 10 Sekunden, so daß das erhaltene Produkt einen Gehalt von Oxiden von etwa 60 bis etwa 80 Gew.-% aufweist,

i) thermische Aktivierung der aus Stufe (h) erhaltenen, trockenen, im wesentlichen homogenen Mischung aus Oxiden bei einer Temperatur zwischen etwa 250 und 500°C.

3. Verfahren zur Herstellung eines Katalysators nach Anspruch 1 oder 2, dadurch gekennzeichnet, daß die Verweilzeit des Präzipitates in den Mutterlaugen in Stufe (a) kürzer als etwa 12 Minuten ist.

4. Verfahren nach einem der Ansprüche 1 bis 3, dadurch gekennzeichnet, daß die Mischung aus Oxiden, die im Verlauf der Stufe (e) in Suspension gebracht wird, eine Teilchengröße von weniger als 0,05 mm aufweist und daß man eine Suspension aus Oxiden bildet, die Oxide von etwa 1,5 bis 6 Gew.-% enthält.

5. Verfahren nach einem der Ansprüche 1 bis 4, dadurch gekennzeichnet, daß im Verlauf der Stufe (f) das Waschen der Suspension aus Oxiden mittels einer verdünnten Salpetersäure, die einen pH-Wert zwischen etwa 2 bis 5 aufweist, durchgeführt wird bis ein Alkalimetallgehalt zwischen etwa 0,005 und etwa 0,035 Gew.-%, bezogen auf die Metalle, erreicht wird, und während der Stufe (h) die Sprühtrocknung während einer Dauer zwischen etwa 1 und 5 Sekunden durchgeführt wird.

6. Verfahren nach einem der Ansprüche 1 bis 5, dadurch gekennzeichnet, daß ein oder mehrere zusätzliche Metalle, ausgewählt aus der Gruppe, gebildet durch die Seltenen Erden, Zirconium, Silber, Palladium und Rhenium in den Katalysator während der Stufe (a) eingebracht werden.

7. Verfahren nach einem der Ansprüche 1 bis 6, dadurch gekennzeichnet, daß ein oder mehrere zusätzliche Metalle, ausgewählt aus der Gruppe, gebildet durch Palladium, Platin, Rhenium und Silber durch Imprägnierung der Mischung aus Oxiden, erhalten in Stufe (i) mit der Hilfe von Salzen und/oder Komplexen dieser Metalle, die weder Schwefel noch Halogene in ihrer Zusammensetzung enthalten, eingebracht werden, der Katalysator dann durch Sprühtrocknung bei einer Temperatur von etwa 60 bis 150°C getrocknet und dann bei einer Temperatur zwischen etwa 250 und 500°C thermisch reaktiviert.

8. Verfahren nach einem der Ansprüche 1 bis 7, dadurch gekennzeichnet, daß die Mengen der Salze oder der Komplexe der verwendeten Metalle zur Herstellung des Katalysators derart gewählt sind, daß die Gewichtsanteile jeden Metallelementes, bezogen auf das Gesamtgewicht der im Katalysator vorhandenen Metalle folgendes ist:

```
Kupfer               = 25-80 %
Zink                 = 10-50 %
Aluminium            =  4-25 %
Seltene Erden
und/oder Zirkonium   = 0-20 %
Palladium            = 0- 1 %
Platin               = 0- 1 %
Rhenium              = 0- 1 %
Silber               = 0- 5 %
Alkalimetalle        = 0- 0,05 %.
```

9. Katalysator im wesentlichen homogener Zusammensetzung, der wenigstens drei wesentliche Elemente enthält: Kupfer, Zink und Aluminium, erhalten nach einem der Ansprüche 1 - 8, wobei die Homogenität derart ist, daß die Änderungen der Atomverhältnisse Cu/Al und Cu/Zn kleiner sind als etwa 15%, bezogen auf den mittleren Wert dieser Verhältnisse im Bereich von 50 Å (5nm), wobei der Katalysator über eine spezifische Oberfläche von etwa 50 bis 150 $m^2 \cdot g^{-1}$ verfügt.

10. Katalysator nach Anspruch 9, dadurch gekennzeichnet, daß er mindestens ein Metall, ausgewählt aus der Gruppe, bestehend aus den Seltenen Erden, Zirkonium, Palladium, Rhenium und Silber, enthält, wobei die Gewichtsanteile jedes vorhandenen Metalls, bezogen auf das Gesamtgewicht der vorhandenen Metalle, folgende sind:

| | | |
|---|---|---|
| Zirkonium | = 2 | – 18 % |
| Seltene Erden | = 2 | – 18 % |
| Palladium | = 0,01 | – 0,9 |
| Rhenium | = 0,01 | – 0,9 % |
| Silber | = 0,05 | – 5 % |

11. Verwendung eines Katalysators nach einem der Ansprüche 9 oder 10 in Verfahren, in denen eine Gleichgewichtsreaktion stattfindet, die auf der einen Seite Kohlenstoffoxide (CO, $CO_2$ und Wasserstoff und auf der anderen Seite einen Alkohol umfaßt.

12. Verwendung nach Anspruch 11, dadurch gekennzeichnet, daß Methanol ausgehend von einer Mischung aus Oxiden von Kohlenstoff (CO, $CO_2$) und Wasserstoff bei einer Temperatur von etwa 200 bis 400°C unter einem Druck im Bereich von 2 bis 15 MPa gebildet wird, wobei das Molverhältnis $H_2/2CO + 3CO_2$ zwischen etwa 0,4 und 10 liegt.

13. Verwendung nach Anspruch 11, dadurch gekennzeichnet, daß man einen primären Alkohol in eine Mischung, die Oxide aus Kohlenstoff (CO, $CO_2$) und Wasserstoff enthält, bei einer Temperatur zwischen etwa 200 bis 320°C unter einem Druck von etwa 1 bis 6 MPa zersetzt.

**Claims**

1. A process for manufacturing a catalyst of essentially homogeneous composition containing at least three essential elements: copper, zinc and aluminium, characterised in that it comprises the following steps:
   a) contacting a solution of at least one alkali metal compound selected from the group formed by hydrogenocarbonates, carbonates and hydroxides with an essentially homogeneous solution containing in the form of soluble complexes and/or salts at least the whole of the metals copper, zinc and aluminium, said contacting operation being performed at a regulated pH of between about 6.3 and 7.3, so as to form an amorphous homogeneous precipitate having a flat X-ray diffraction diagram of at least the metals copper, zinc and aluminium, the residence time of said precipitate in the mother liquors being from 0.1 to 30 minutes,
   b) washing with water the precipitate obtained in step (a) until its content of alkali metals is reduced to a value of between about 0.1 and 0.3 % by weight with respect to the metals expressed by weight,
   c) drying and then roasting of the washed precipitate obtained in step (b) so as to convert it into a mixture of oxides,
   d) crushing said mixture of oxides to a granulometry of less than about 0. 5 millimetre when the mixture of oxides obtained in step (c) is of a granulometry of greater than 0.5 millimetre,
   e) forming an aqueous suspension of the mixture of oxides of a granulometry of smaller than about 0.5 millimetre,
   f) washing the suspension of oxides obtained in step (e) by means of an aqueous solution of an acid, said solution having a pH lower than about 6.7, so as to reduce its content of alkali metals to a value of less than about 0.05% by weight with respect to the whole of the metals,
   g) separating the dealkalinized mixture of oxides obtained in step (f) from the washing waters,
   h) spray-drying the dealkalinized mixture of oxides obtained in step (g) at a temperature of from about 60 to 150°C with a residence time of between 1 and 10 seconds, so as to obtain a product having an oxides content of from about 60% to about 80% by weight, and
   i) thermally activating the essentially homogeneous dried mixture of oxides obtained in step (h).

2. A process for manufacturing a catalyst according to claim 1 characterised in that it comprises the following steps:
   a) contacting a solution of at least one alkali metal compound selected from the group formed by hydrogenocarbonates, carbonates and hydroxides, containing from about 0.4 to about 1.2 gram atoms per litre of cations of alkali metals, with an essentially homogeneous solution containing in the form of soluble complexes and/or salts at least the whole of the metals copper, zinc and aluminium at a total concentration of from about 0.2 to about 0.6 gram atom per litre, said contacting operation being per-

18

formed at a pH of between about 6.3 and 7.3 and at a temperature of between about 20 and 80°C, so as to form an amorphous homogeneous precipitate having a flat X-ray diffraction diagram of at least the metals copper , zinc and aluminium, the residence time of said precipitate in the mother liquors being from 0.1 to 30 minutes,

b) washing the precipitate obtained until its content of alkali metals is reduced to a value of between about 0.05 and about 0.2% by weight with respect to the metals expressed by weight,

c) drying and then roasting the precipitate obtained in step (b) so as to convert it into a mixture of oxides, the roasting operation being performed at a temperature of between about 250° and 500°C,

d) crushing said mixture of oxides to a granulometry of smaller than about 0.5 millimetre when the mixture of oxides obtained in step (c) is of a granulometry of larger than about 0.5 millimetre,

e) forming an aqueous suspension containing from about 0.5 to about 10% by weight of oxides of the mixture of oxides of a granulometry of smaller than about 0.5 mm,

f) washing the suspension of oxides obtained in step (e) by means of an aqueous solution of an acid, said solution having a pH of lower than about 6, at a temperature of from about 15 to 45°C, so as to reduce the content of alkali metals to a value of lower than about 0.05% by weight with respect to the metals,

g) separating the dealkalinized mixture of oxides obtained in step (f) from the washing waters,

h) spray-drying the dealkalinized mixture of oxides obtained in step (g) at a temperature of from about 60 to 150°C with a residence time of between about 1 and 10 seconds so as to obtain a product having an oxides content of from about 60% to about 80% by weight, and

i) thermally activating the essentially homogeneous dry mixture of oxides obtained in step (h) at a temperature of between about 250 and 500°C.

3. A process for manufacturing a catalyst according to claim 1 or claim 2 characterised in that the residence time of the precipitate in the mother liquors in step (a) is less than about 12 minutes.

4. A process according to any one of claims 1 to 3 characterised in that the mixture of oxides put into suspension in the course of step (e) is of a granulometry of smaller than about 0.05 mm and a suspension of oxides containing from about 1.5 to 6% by weight of oxides is formed.

5. A process according to any one of claims 1 to 4 characterised in that, in the course of step (f), washing of the suspension of oxides is performed by means of a dilute solution of nitric acid having a pH of between about 2 and 5 until a content of alkali metals of between about 0.005 and about 0.035% by weight with respect to the metals is obtained and, in the course of step (h), the drying operation is effected by spray-drying for a period of between about 1 and 5 seconds.

6. A process according to any one of claims 1 to 5 characterised in that one or more additional metals selected from the group formed by rare earths, zirconium, silver, palladium and rhenium, are introduced into the catalyst in the course of step (a).

7. A process according to any one of claims 1 to 6 characterised in that one or more additional metals selected from the group formed by palladium, platinum, rhenium and silver are introduced by impregnating the mixture of oxides obtained in step (i) by means of salts and/or complexes of those metals, which do not contain sulphur or halogen in their compositions, the catalyst then being spray-dried at a temperature of from about 60 to 150°C and then thermally reactivated at a temperature of between about 250 and 500°C.

8. A process according to one of claims 1 to 7 characterised in that the amounts of salts or complexes of the metals used for the formation of the catalyst are such that the proportions by weight of each metallic element with respect to the total weight of the metals present in the catalyst are:

Copper = 25-80%
Zinc = 10-50%
Aluminium = 4-25%
Rare earths and/or zirconium = 0-20%
Palladium = 0-1%
Platinum = 0-1%
Rhenium = 0-1%
Silver = 0-5%
Alkali metals = 0-0.05%.

9. A catalyst of essentially homogeneous composition containing at least three essential elements, copper, zinc and aluminium, obtained in accordance with one of claims 1 to 8, the homogeneity being such that the variations in the atomic ratios Cu/Al and Cu/Zn are less than about 15% with respect to the average value of said ratios, at the scale of 50 Angströms (5 nm), the specific surface area of the catalyst being from about 50 to 150 $m^2.g^{-1}$.

10. A catalyst according to claim 9 characterised in that it contains at least one metal selected from the group formed by rare earths, zirconium, palladium, rhenium and silver, the proportions by weight of each metal present with respect to the total weight of the metals present being:

Zirconium = 2-18%
Rare earths = 2-18%
Palladium = 0.01-0.9%
Rhenium = 0.01-0.9%
Silver = 0.5-5%.

11. Use of a catalyst according to one of claims 9 and 10 in processes involving a balanced reaction comprising on the one hand carbon oxides (CO, $CO_2$) and hydrogen and on the other hand an alcohol.

12. Use according to claim 11 characterised in that methanol is formed from the mixture of carbon oxides (CO, $CO_2$) and hydrogen at a temperature of from about 200 to 400°C under a pressure of from about 2 to 15 MPa, the molar ratio $H_2/2CO + 3CO_2$ being between about 0.4 and 10.

13. Use according to claim 11 characterised in that a primary alcohol is decomposed into a mixture containing carbon oxides (CO, $CO_2$) and hydrogen at a temperature of from about 200 to 320°C under a pressure of from about 1 to 6 MPa.